# EUROPEAN PATENT SPECIFICATION

(11) **EP 1 587 463 B1**
(45) Date of publication and mention of the grant of the patent: **14.03.2007**
(21) Application number: 03703588.8
(22) Date of filing: 31.01.2003
(51) Int. Cl.: A61F 2/48, A61F 5/48

(54) **ELECTRICALLY OPERABLE INCONTINENCE TREATMENT APPARATUS**
ELEKTRISCH BEDIENBARE VORRICHTUNG ZUR INKONTINENZBEHANDLUNG
APPAREIL ELECTRIQUE DE TRAITEMENT DE L'INCONTINENCE

(43) Date of publication of application: 26.10.2005
(73) Proprietor: Potencia Medical AG, 6341 Baar (CH)
(72) Inventor: FORSELL, Peter, CH-6300 Zug (CH)
(74) Representative: Strandin, Heléne
(86) International application number: PCT/SE2003/000170
(87) International publication number: WO 2004/066885

(56) References cited:
- WO-A1-00/15158
- WO-A1-01/12078
- WO-A2-01/47431
- FR-A1- 2 688 693

## Description

The present invention relates to an apparatus for treating an anal or urinary incontinent patient comprising a constriction device adapted to be applied on the patient's large intestine or urethra, and an electrically powered operation device that operates the constriction device to constrict the large intestine or urethra to close the intestinal or urethral lumen and to release the large intestine or urethra to open the intestinal or urethral lumen. The apparatus further comprises an electric power supply adapted to be implanted in the patient remote from the operation device, and an insulated electric wire connecting the electric power supply and the operation device The term "patient" includes an animal or a human being. The term "large intestine" includes the colon, rectum and anal canal.

Anal incontinence is a widespread disease. Several kinds of sphincter plastic surgery are used today to remedy anal incontinence. There is a prior manually operated sphincter system in an initial clinical trial phase where a hydraulic sphincter system connected to an elastic reservoir (balloon) placed in the scrotum is developed. A disadvantage of this system is that thick, hard fibrosis is created around the reservoir by pump movements making the system useless sooner or later.

U.S. Pat. No. 5593443 discloses a hydraulic anal sphincter under both reflex and voluntary control. A pressure controlled inflatable artificial sphincter is disclosed in U.S. Pat. No. 4222377.

Also urinary incontinence is a widespread problem. Many people are helped through training of the muscles in the pelvic floor but too many have severe problems with urine leakage. Many different solutions to this problem have been tried. For example, there is a prior manually operated urine incontinence treatment apparatus having an artificial hydraulic sphincter device engaging the urethra and connected to an elastic reservoir implanted in the scrotum or in the region of the labia major. A disadvantage of this prior apparatus is that over time hard fibrosis is developed around the reservoir, which may cause malfunction of pumping components. Furthermore, it is a rather complicated task to manually squeeze the elastic implanted reservoir to pump hydraulic fluid to open the sphincter device when the patient needs to urinate. In particular women can get their fingers wet. The created fibrosis will sooner or later become a hard fibroid layer, which may make it even more difficult to pump the reservoir. Yet a further disadvantage is that the use of hydraulic fluid always entails a risk of fluid leaking from implanted hydraulic components.

A prior hydraulic apparatus designed to constrict the urethra is disclosed in U.S. Pat. No. 5520606. A prosthetic sphincter with an inflatable cuff, which surrounds the urethra or encloses it on two sides, is disclosed in for example U.S. Pat. Nos. 4571749 and 4222377. U.S. Pat. No. 4969474 discloses a hydraulic method for treating both men and women with urinary incontinence problems in the same way. The apparatus of U.S. Pat. No. 4969474 includes a reservoir containing fluid and an inflatable compression means designed to constrict the urethra without risking tissue loss or necrosis to occur. An artificial hydraulically operated urethra sphincter employing an external magnet to achieve closure of the urethra cuff is disclosed in U.S. Pat. No. 5562598.

A prior mechanical prosthetic sphincter disclosed in U.S. Pat. No. 4619245 comprises a manually controllable actuating component for implanting at a convenient location in the patient's body.

A mechanical apparatus for constricting the large intestine or urethra may typically involve an electrically powered operation device connected to the constriction device. Therefore, such an operation device would be located at the patient's large intestine or urethra. On the other hand, the electric power supply, for example a battery, necessary for powering the operation device with electric energy should be implanted subcutaneously, i.e. remote from the operation device, in order to permit easy access from outside the patient's body for service or replacement of the power supply. In consequence, it is necessary to implant a fairly long electric wire for connecting the power supply to the operation device. However, considering the fact that the apparatus is to be implanted for many years, possibly for the rest of the patient's life, the use of such an electric wire would make the apparatus unreliable, because the movements of the large intestine or urethra might sooner or later break the electric wire.

The object of the present invention is to provide a new reliable electrically operable apparatus for constricting the large intestine or urethra of an incontinent patient.

This object is obtained by an apparatus of the kind presented initially characterised in that the electric wire is resilient and extends helically between the electric power supply and the electrically powered operation device. As a result, the resilient helically extending electric wire is capable of addressing the movements from the large intestine or urethra, because it may easily be temporarily extended without risking breakage.

The electric wire should be designed to conduct a current in the order of milliamperes, which is needed for powering the operation device when operating the constriction device, so that the constriction device exerts a force strong enough to constrict the large intestine or uretrhra. Thus, the electric wire should have a low resistance.

Advantageously, the apparatus may include an energy transforming device capable of transforming wireless energy transmitted from outside the patient's body into electric energy, whereby the need for regularly replacing the electric power supply, when it is depleted, is eliminated.

Preferably, the operation device includes an electric motor connected to the electric power supply by means of the helical electrical wire. The constriction device may include a hydraulic constriction device inflatable by hydraulic fluid, and the operation device may include an electrically driven pump (for example driven by the electric motor) hydraulically connected to the hydraulic constriction device.

In accordance with a preferred embodiment of the invention, the constriction device is non-inflatable. In this embodiment, the constriction device comprises an elongate constriction member adapted to extend around the large intestine or urethra. The constriction member includes a main portion and two elongated end portions, and the operation device establishes longitudinal relative displacement between the end portions of the constriction member to constrict or release the large intestine or urethra. The operation device may include a movement transferring means in engagement with at least one of the end portions of the constriction member and operable by the electric motor to displace the one end portion relative to the other end portion of the constriction member. The movement transferring means may include a gear wheel fixed to the other end portion of the constriction member and a gear rack formed on the one end portion of the constriction member, the gear wheel and the gear rack being in mesh with each other. The operation device may further include a worm gear connected between the electric motor and the gear wheel.

Generally, the constriction device includes a housing containing the operation device, and the elongate constriction member and the housing are adapted to form a loop around the patient's large intestine or urethra.

Advantageously, the apparatus includes a control device, suitably in the form of a wireless remote control, for controlling the operation device to operate the constriction device. The control device may include an internal, preferably programmable, control unit implantable in the patient for controlling the operation device. Furthermore, the control device may include an external control unit outside the patient's body, wherein the internal control unit is programmable by the external control unit.

The apparatus may include at least one implantable sensor for sensing at least one physical parameter of the patient. The sensor may be a pressure sensor for directly or indirectly sensing the pressure against the constriction device. The control device may suitably control the operation device in response to signals from the sensor. Where the control device includes an internal control unit, the internal control unit may control the operation device in response to signals from the sensor.

The apparatus may include a switch implantable in the patient for directly or indirectly switching the electric energy supplied by the electric power supply.

### In the enclosed drawings:

Figure 1 is a schematic view of an apparatus according to the present invention implanted in an incontinent patient, and

Figure 2 schematically illustrates an embodiment of a constriction device of the apparatus for use in the embodiment of Figure 1.

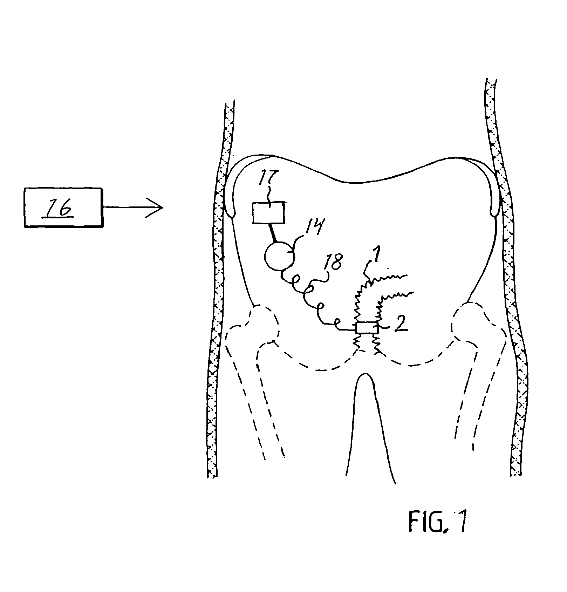

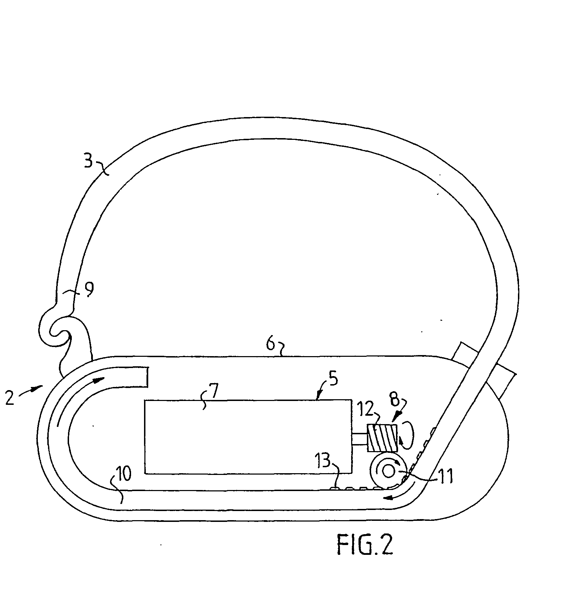

Figure 1 shows an apparatus of the present invention for treating an anal incontinent patient comprising a constriction device 2 applied on the rectum 1. The constriction device 2 includes an elongate operable constriction member 3 extending in a loop around and constricting the rectum 1 to normally close the rectal lumen. An electrically powered operation device 5 that operates the constriction member 3 to open and close the rectal lumen is housed in an elongate housing 6, see Figure 2. The constriction member 3 has a first end portion 9 releasably connected to the housing 6 and a second end portion 10 connected to the operation device 5. The operation device 5 includes an electric motor 7 and a movement transferring means 8 in engagement with the end portion 10. The electric motor 7 operates the movement transferring means 8 to displace the end portion 10 relative to portion 9 in the loop formed by the constriction member 3. The movement transferring means 8 includes a gear wheel 11 fixed to the housing 6, a worm gear 12 connected between the electric motor 7 and the gear wheel 11, and a gear rack 13 formed on the end portion 10, wherein the gear wheel 11 and the gear rack 13 are in mesh with each other.

A rechargeable electric power supply 14 is subcutaneously implanted in the patient. An external remote control 16 controls the operation device 5 and transmits signals that are received by a combined control and energy transforming unit 17 subcutaneously implanted in the patient. The unit 17 is electrically connected to the electric power supply 14 and transforms the energy of the signals into an electric current that is used for charging the electric power supply 14. For example, the signals may include electromagnetic waves and the unit 17 may include an electric p-n junction element that transforms the wireless energy into an electric current.

A resilient insulated electric wire 18 connects the power supply 14 and the electric motor 7. The electric wire 18 extends helically between the power supply 14 and housing 6 that contains the motor 7, in order to permit the electric wire 18 to be temporarily extended when movements of the rectum occur, so that the risk of breaking the electric wire 18 is eliminated.

## Claims

1. An apparatus for treating an anal or urinary incontinent patient comprises a constriction device (2) adapted to be applied on the patient's large intestine or urethra, an electrically powered operation device (5) that operates the constriction device to constrict the large intestine or urethra to close the intestinal or urethral lumen and to release the large intestine or urethra to open the intestinal or urethral lumen, an electric power supply (14) adapted to be implanted in the patient remote from the operation device, and an insulated electric wire (18) connecting the electric power supply and the operation device, **characterized in that** the electric wire (18) is resilient and extends helically between the electric power supply (14) and the electrically powered operation device (5).

2. An apparatus according to claim 1, further comprising an implantable energy transforming device (17) capable of transforming wireless energy transmitted from outside the patient's body into electric energy.

3. An apparatus according to claim 1 or 2, wherein the operation device (5) comprises an electric motor (7) connected to the electric power supply (14) by means of the helical electrical wire (18).

4. An apparatus according to claim 3, wherein the constriction device (2) is non-inflatable.

5. An apparatus according to claim 4, wherein the constriction device (2) comprises an elongate constriction member (3) adapted to extend around the large intestine or urethra.

6. An apparatus according to claim 5, wherein the constriction member (3) comprises a main portion and two elongated end portions (9,10), and the operation device (5) establishes longitudinal relative displacement between the end portions of the constriction member.

7. An apparatus according to claim 6, wherein the operation device (5) comprises an electric motor and a movement transferring means (8) in engagement with at least one (10) of the end portions of the constriction member (3) and operable by the electric motor (7) to displace said one end portion (10) relative to the other end portion (9) of the constriction member.

8. An apparatus according to claim 7, wherein the movement transferring means (8) comprises a gear wheel (11) fixed relative to said other end portion (9) of the constriction member (3) and a gear rack (13) formed on said one end portion (10) of the constriction member, the gear wheel and the gear rack being in mesh with each other.

9. An apparatus according to claim 8, wherein the operation device (5) comprises a worm gear (12) connected between the electric motor (7) and the gear wheel (11).

10. An apparatus according to any one of claims 1-3, wherein the constriction device (2) comprises a hydraulic constriction device and the operation device comprises an electrically driven pump hydraulically connected to the hydraulic constriction device.

11. An apparatus according to any one of claims 5-9, wherein the constriction device (2) comprises a housing (6) containing the operation device (5), and the elongate constriction member (3) and the housing are adapted to form a loop around the patient's large intestine or urethra.

12. An apparatus according to any one of claims 1-11, further comprising a wireless remote control (16) for controlling the operation device (5) to operate the constriction device (2).

13. An apparatus according to any one of claims 1-12, further comprising at least one implantable sensor for sensing at least one physical parameter of the patient.

14. An apparatus according to claim 13, wherein the sensor comprises a pressure sensor for directly or indirectly sensing the pressure against the constriction device (2).

15. An apparatus according to claim 14, further comprising a control device for controlling the operation device in response to signals from the sensor.

16. An apparatus according to claim 15, wherein the control device comprises an internal control unit implantable in the patient for controlling the operation device in response to signals from the sensor.

17. An apparatus according to any one of claims 1-14, further comprising a control device for controlling the operation device

18. An apparatus according to claim 17, wherein the control device comprises an internal control unit implantable in the patient for controlling the operation device.

19. An apparatus according to claim 18, wherein the internal control unit is programmable.

20. An apparatus according to claim 19, wherein the control device comprises an external control unit outside the patient's body, the internal control unit being programmable by the external control unit.

21. An apparatus according to any one of claims 1-20, further comprising a switch implantable in the patient for directly or indirectly switching the electric energy supplied by the electric power supply.

## Patentansprüche

1. Vorrichtung zur Behandlung eines anal oder urinal inkontinenten Patienten mit einer Konstriktionsvorrichtung (2), die derart ausgebildet ist, dass sie an dem Dickdarm oder dem Harnleiter des Patienten angelegt werden kann, einer elektrisch betriebenen Betätigungsvorrichtung (5), welche die Konstriktionsvorrichtung zum Einschnüren des Dickdarms oder des Harnleiters betätigt, um das Darm- oder Harnleiterlumen zu schließen, einer elektrischen Energiequelle (14), die von der Betätigungsvorrichtung entfernt in den Patienten implantierbar ist, und einem isolierten elektrischen Draht (18), welcher die elektrische Energiequelle und die Betätigungsvorrichtung miteinander verbindet, **dadurch gekennzeichnet, dass** der elektrische Draht (18) elastisch ist und sich schraubenlinienförmig zwischen der elektrischen Energiequelle (14) und der elektrisch betriebenen Betätigungsvorrichtung (5) erstreckt.

2. Vorrichtung nach Anspruch 1, ferner mit einer implantierbaren Energieumwandelvorrichtung (17), welche derart ausgebildet ist, dass sie von außerhalb des Patientenkörpers zugeführte drahtlose Energie in elektrische Energie umwandelt.

3. Vorrichtung nach Anspruch 1 oder 2, bei der die Betätigungsvorrichtung (5) einen Elektromotor (7) aufweist, der mit der elektrischen Energiequelle (14) durch den schraubenlinienförmigen elektrischen Draht (18) verbunden ist.

4. Vorrichtung nach Anspruch 3, bei der die Konstriktionsvorrichtung (2) nicht aufblasbar ist.

5. Vorrichtung nach Anspruch 4, bei der die Konstriktionsvorrichtung (2) ein langgestrecktes Konstriktionselement (3) aufweist, das sich um den Dickdarm oder den Harnleiter herum erstreckt.

6. Vorrichtung nach Anspruch 5, bei der das Konstriktionselement (3) einen Hauptbereich und zwei langgestreckte Endbereiche (9, 10) aufweist, und die Betätigungsvorrichtung (5) eine relative Längsverschiebung zwischen den Endbereichen des Konstriktionsteils bewirkt.

7. Vorrichtung nach Anspruch 6, bei der die Betätigungsvorrichtung (5) einen Elektromotor und eine Bewegungsübertragungseinrichtung (8) aufweist, welche in Eingriff mit mindestens einem (10) der Endbereiche des Konstriktionselements (3) steht und von dem Elektromotor (7) zum Verschieben des einen Endbereichs (10) in bezug auf den anderen Endbereich (9) des Konstriktionselements betätigbar ist.

8. Vorrichtung nach Anspruch 7, bei der die Bewegungsübertragungseinrichtung (8) ein in bezug auf den anderen Endbereich (9) des Konstriktionselements (3) angebrachtes Zahnrad (11) und eine an dem einen Endbereich (10) des Konstriktionselements ausgebildete Zahnstange (13) aufweist, wobei das Zahnrad und die Zahnstange miteinander kämmen.

9. Vorrichtung nach Anspruch 8, bei der die Betätigungsvorrichtung (5) ein Schneckenrad (12) aufweist, das zwischen dem Elektromotor (7) und dem Zahnrad (11) angeordnet ist.

10. Vorrichtung nach einem der Ansprüche 1 - 3, bei der die Konstriktionsvorrichtung (2) eine hydraulische Konstriktionsvorrichtung und die Betätigungseinrichtung eine elektrisch angetriebene Pumpe aufweist, die hydraulisch mit der hydraulischen Konstriktionsvorrichtung verbunden ist.

11. Vorrichtung nach einem der Ansprüche 5-9, bei der die Konstriktionsvorrichtung (2) ein Gehäuse (6) aufweist, welches die Betätigungsvorrichtung (5) enthält, und das langgestreckte Konstriktionselement (3) und das Gehäuse zur Bildung einer Schlaufe um den Dickdarm oder den Harnleiter eines Patienten ausgebildet sind.

12. Vorrichtung nach einem der Ansprüche 1-11, ferner mit einer drahtlosen Fernsteuerung (16) zum Steuern der Betätigungsvorrichtung (5) zum Betreiben der Konstriktionsvorrichtung (2).

13. Vorrichtung nach einem der Ansprüche 1-12, ferner mit mindestens einem implantierbaren Sensor zum Erkennen mindestens eines physischen Parameters des Patienten.

14. Vorrichtung nach Anspruch 13, bei welcher der Sensor einen Drucksensor zum unmittelbaren oder mittelbaren Erkennen des gegen die Konstriktionsvorrichtung (2) wirkenden Drucks aufweist.

15. Vorrichtung nach Anspruch 14, ferner mit einer Steuervorrichtung zum Steuern der Betätigungsvorrichtung als Antwort auf Signale des Sensors.

16. Vorrichtung nach Anspruch 13, bei der die Steuervorrichtung eine interne Steuereinheit aufweist, die in den Patienten implantierbar ist, um die Betätigungsvorrichtung als Antwort auf Signale des Sensors zu steuern.

17. Vorrichtung nach einem der Ansprüche 1-14, ferner mit einer Steuervorrichtung zum Steuern der Betätigungsvorrichtung.

18. Vorrichtung nach Anspruch 17, bei der die Steuervorrichtung eine interne Steuereinheit aufweist, die in den Patienten implantierbar ist, um die Betätigungsvorrichtung zu steuern.

19. Vorrichtung nach Anspruch 18, bei der die interne Steuereinheit programmierbar ist.

20. Vorrichtung nach Anspruch 19, bei der die Steuervorrichtung eine externe Steuereinheit außerhalb des Patientenkörpers aufweist, wobei die innere Steuereinheit durch die externe Steuereinheit programmierbar ist.

21. Vorrichtung nach einem der Ansprüche 1-20, ferner mit einem in den Patienten implantierbaren Schalter zum unmittelbaren oder mittelbaren Schalten der von der elektrischen Energiequelle gelieferten elektrischen Energie.

## Revendications

1. Appareil de traitement d'un patient ayant une incontinence anale ou urinaire, comprenant un dispositif (2) de rétrécissement destiné à être appliqué sur le gros intestin ou l'urètre du patient, un dispositif électrique de manoeuvre (5) qui agit sur le dispositif de rétrécissement afin qu'il rétrécisse le gros intestin ou l'urètre et ferme la lumière intestinale ou urétrale et libère le gros intestin ou l'urètre pour ouvrir la lumière intestinale ou urétrale, une alimentation électrique (14) destinée à être implantée dans le patient à distance du dispositif de manoeuvre, et un fil électrique isolé (18) reliant l'alimentation électrique au dispositif de manoeuvre, **caractérisé en ce que** le fil électrique (18) est élastique et s'étend en hélice entre l'alimentation électrique (14) et le dispositif électrique de manoeuvre (5).

2. Appareil selon la revendication 1, comprenant en outre un dispositif implantable (17) de transformation d'énergie capable de transformer l'énergie sans fil transmise depuis l'extérieur du corps du patient en énergie électrique.

3. Appareil selon la revendication 1 ou 2, dans lequel le dispositif de manoeuvre (5) comporte un moteur électrique (7) connecté à l'alimentation électrique (14) par le fil électrique en hélice (18).

4. Appareil selon la revendication 3, dans lequel le dispositif de rétrécissement (2) n'est pas gonflable.

5. Appareil selon la revendication 4, dans lequel le dispositif de rétrécissement (2) comprend un organe allongé (3) de rétrécissement destiné à s'étendre autour du gros intestin ou de l'urètre.

6. Appareil selon la revendication 5, dans lequel l'organe de rétrécissement (3) comporte une partie principale et deux parties allongées d'extrémité (9, 10), et le dispositif de manoeuvre (5) établit un déplacement relatif longitudinal entre les parties d'extrémité de l'organe de rétrécissement.

7. Appareil selon la revendication 6, dans lequel le dispositif de manoeuvre (5) comprend un moteur électrique et un dispositif (8) de transfert de mouvement en coopération avec l'une au moins (10) des parties d'extrémité de l'organe de rétrécissement (3) et destiné à être manoeuvré par le moteur électrique (7) pour déplacer cette partie d'extrémité (10) par rapport à l'autre partie d'extrémité (9) de l'organe de rétrécissement.

8. Appareil selon la revendication 7, dans lequel le dispositif (8) de transfert de mouvement comprend une roue dentée (11) fixée par rapport à l'autre partie d'extrémité (9) de l'organe de rétrécissement (3), et une crémaillère (13) formée sur la première partie d'extrémité (10) de l'organe de rétrécissement, la roue dentée et la crémaillère étant en prise mutuellement.

9. Appareil selon la revendication 8, dans lequel le dispositif de manoeuvre (5) comprend une vis d'engrenage (12) raccordée entre le moteur électrique (7) et la roue dentée (11).

10. Appareil selon l'une quelconque des revendications 1 à 3, dans lequel le dispositif de rétrécissement (2) comprend un dispositif hydraulique de rétrécissement et le dispositif de manoeuvre comprend une pompe électrique raccordée hydrauliquement au dispositif hydraulique de rétrécissement.

11. Appareil selon l'une quelconque des revendications 5 à 9, dans lequel le dispositif de rétrécissement (2) comprend un boîtier (6) contenant le dispositif de manoeuvre (5), et l'organe allongé de rétrécissement (3) et le boîtier sont destinés à former une boucle autour du gros intestin ou de l'urètre du patient.

12. Appareil selon l'une quelconque des revendications 1 à 11, comprenant en outre une commande à distance sans fil (16) destinée à commander le dispositif de manoeuvre (5) pour la manoeuvre du dispositif de rétrécissement (2).

13. Appareil selon l'une quelconque des revendications 1 à 12, comprenant en outre au moins un capteur implantable destiné à détecter au moins un paramètre physique du patient.

14. Appareil selon la revendication 13, dans lequel le capteur comprend un capteur de pression destiné à détecter directement ou indirectement la pression exercée contre le dispositif de rétrécissement (2).

15. Appareil selon la revendication 14, comprenant en outre un dispositif de commande du dispositif de manoeuvre en fonction de signaux provenant du capteur.

16. Appareil selon la revendication 15, dans lequel le dispositif de commande comprend une unité interne de commande implantable dans le patient pour la commande du dispositif de manoeuvre en fonction de signaux provenant du capteur.

17. Appareil selon l'une quelconque des revendications 1 à 14, comprenant en outre un dispositif de commande de dispositif de manoeuvre.

18. Appareil selon la revendication 17, dans lequel le dispositif de commande comprend une unité interne de commande implantable dans le patient pour la commande du dispositif de manoeuvre.

19. Appareil selon la revendication 18, dans lequel l'unité interne de commande est programmable.

20. Appareil selon la revendication 19, dans lequel le dispositif de commande comprend une unité externe de commande placée à l'extérieur du corps du patient, l'unité interne de commande étant programmable par l'unité externe de commande.

21. Appareil selon l'une quelconque des revendications 1 à 20, comprenant en outre un interrupteur implantable dans le patient et destiné à commuter directement ou indirectement l'énergie électrique transmise par l'alimentation électrique.
